# EUROPEAN PATENT SPECIFICATION

(11) **EP 2 878 256 B1**
(45) Date of publication and mention of the grant of the patent: **09.01.2019**
(21) Application number: 14195372.9
(22) Date of filing: 28.11.2014
(51) Int. Cl.: A61B 3/10

(54) **Ophthalmic measurement apparatus**
Ophthalmologische Messvorrichtung
Appareil de mesure ophtalmique

(30) Priority: 29.11.2013 JP 2013248477
(43) Date of publication of application: 03.06.2015
(73) Proprietor: Nidek Co., Ltd., Aichi 443-0038 (JP)
(72) Inventor: Takii, Michihiro, Gamagori-shi, Aichi 4430038 (JP); Kawai, Noriji, Gamagori-shi, Aichi 4430038 (JP); Sakashita, Yusuke, Gamagori-shi, Aichi 4430038 (JP)
(74) Representative: Prüfer & Partner mbB Patentanwälte · Rechtsanwälte

(56) References cited:
- JP-A- 2012 143 492
- US-A1- 2004 021 874
- US-A1- 2011 013 141

## Description

### Field of the Invention

This disclosure relates to an ophthalmic measurement apparatus for measuring corneal thickness of an examinee's eye.

### Related Art

Conventionally, there is known an apparatus for measuring corneal thickness by projecting measurement light to an examinee's eye from an oblique direction and utilizing reflection light of the measurement light from a corneal anterior surface of the examinee's eye and reflection light of the measurement light from a corneal posterior surface of the examinee's eye.

For instance, Patent Document 1 discloses an apparatus for measuring corneal thickness by receiving reflection light from a corneal anterior surface and reflection light from a corneal posterior surface in different positions of a light receiving element such as a one-dimensional line sensor and others, and using a one-dimensional received light intensity distribution representing the intensity of light received in each position on the light receiving element.

### Related Art Documents

### Patent Documents

Patent Document 1: JP-A-2012-143492

### SUMMARY OF INVENTION

### Problems to be Solved by the Invention

Meanwhile, there may be a case where scattered measurement light from a cornea influences reflection light which will enter a light receiving element. For instance, the measurement light may be scattered due to corneal abnormality, extraneous matters sticking to a corneal front surface, and others. Also, scattered measurement light within a cornea may enter the light receiving element. The conventional apparatus is merely arranged to one-dimensionally determine the positions on the anterior surface and the posterior surface of a cornea. This apparatus is not designed to take into consideration the influence of scattered light that will enter the light receiving element.

The present disclosure has been made to solve the above problems and has a purpose to provide an ophthalmic measurement apparatus capable of obtaining a good measurement value of corneal thickness.

### Means of Solving the Problems

To achieve the above purpose, one aspect of this disclosure provides an ophthalmic measurement apparatus comprising: a light projecting optical system for projecting measurement light to an examinee's eye from an oblique direction; a light receiving optical system for receiving, by a light receiving element, a first cornea reflection image formed by the measurement light reflected by a corneal anterior surface of the examinee's eye and a second corneal reflection image formed by the measurement light reflected by a corneal posterior surface of the examinee's eye; and an arithmetic processing unit for obtaining corneal thickness of the examinee's eye based on positional information of the first cornea reflection image and the second cornea reflection image in a light receiving signal output from the light receiving element, characterized in that the light receiving optical system includes a two-dimensional light receiving element as the light receiving element, and the arithmetic processing unit is configured to two-dimensionally detect the positional information of at least one of the first cornea reflection image and the second cornea reflection image based on a light receiving signal from the two-dimensional light receiving element, and obtain the corneal thickness by use of the two-dimensionally detected positional information.

The invention is defined in claim 1. Further developments of the present disclosure are given in the dependent claims.

### Effects of the present disclosure

According to an ophthalmic measurement apparatus of the present disclosure, it is possible to obtain a more preferable measurement value of corneal thickness.

### BRIEF DESCRIPTION OF THE DRAWING

FIG. 1 illustrates a schematic configuration of a measuring unit in an ophthalmic measurement apparatus of a present embodiment;
FIG. 2 is a schematic diagram showing light beams emitted from a corneal thickness measurement optical system and reflected by a cornea;
FIG. 3A is a diagram showing an image captured by use of a light receiving element of the corneal thickness measurement optical system;
FIG. 3B is a diagram to explain an image processing to be performed on the image shown in FIG. 3A;
FIG. 4 is a block diagram showing a schematic configuration of a control system in the ophthalmic measurement apparatus of the present embodiment;
FIG. 5 is a flowchart showing a corneal thickness measurement processing to be executed by a CPU in the ophthalmic measurement apparatus; and
FIG. 6 is a schematic diagram to explain a measurement method in a modified example of the present disclosure.

### DESCRIPTION OF EMBODIMENTS

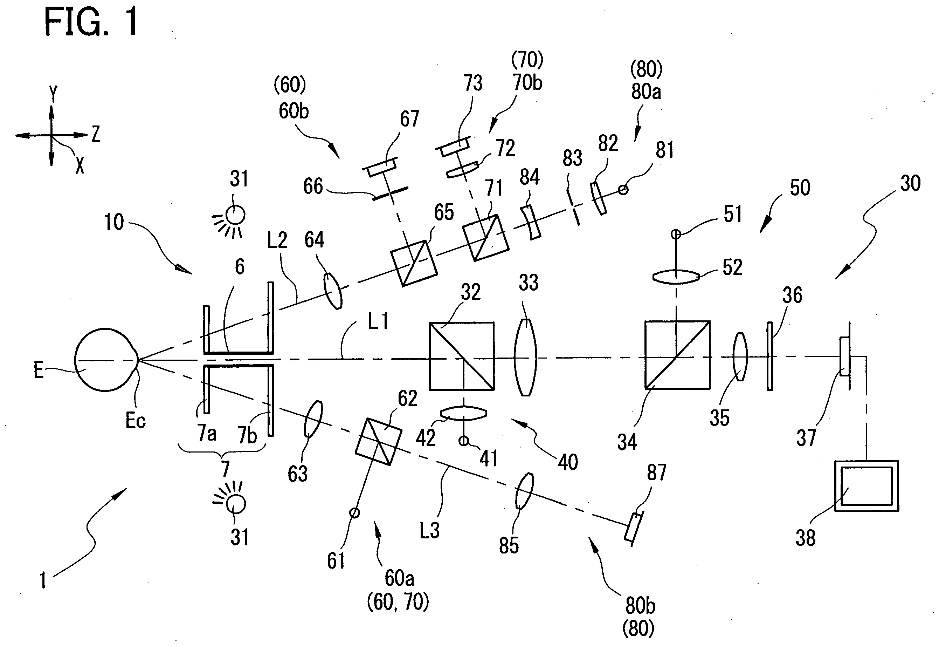

A detailed description of a preferred embodiment of the present disclosure will now be given referring to the accompanying drawings. A schematic configuration of a measuring unit in an ophthalmic measurement apparatus 1 of the present embodiment will be first explained referring to FIG. 1.

The ophthalmic measurement apparatus 1 shown in FIG. 1 is configured to measure corneal thickness of an examinee's eye E. In the present embodiment, the apparatus is arranged to measure intraocular pressure in non-contact manner.

The ophthalmic measurement apparatus 1 mainly includes an observation optical system 30 and a corneal thickness measurement optical system 80 (80a and 80b in FIG. 1). In the present embodiment, the ophthalmic measurement apparatus 1 further includes an alignment optical system 40, a fixation optical system 50, a corneal deformation detecting optical system 60 (60a and 60b in FIG. 1), and a first working distance detecting optical system 70 (60a and 70b in FIG. 1). Further, the ophthalmic measurement apparatus 1 of the present embodiment also includes an intraocular pressure measuring unit 10.

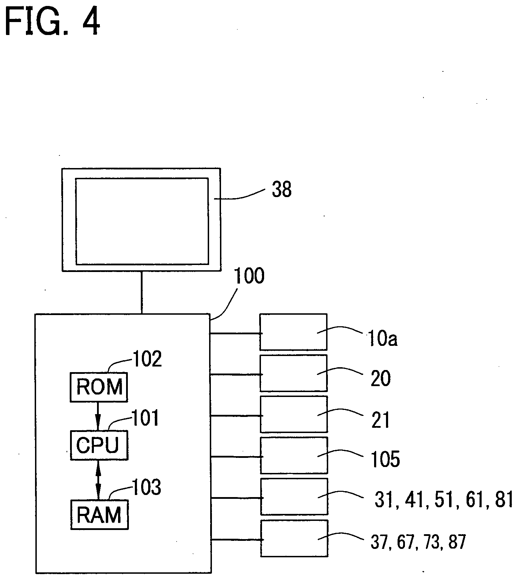

In the present embodiment, the measuring unit of the ophthalmic measurement apparatus 1 including those optical systems and others is housed in a cabinet not shown. The cabinet is three-dimensionally movable with respect to the examinee's eye E by an XYZ drive mechanism 20 (see FIG. 4). This cabinet may also be moved according to an instruction of an examiner for example through an operating member (e.g., an operating unit (a joystick) 21, see FIG. 4).

The observation optical system 30 is used to observe a front image of an anterior segment of the examinee's eye E. The observation optical system 30 includes a beam splitter 32, an objective lens 33, a beam splitter 34, an imaging lens 35, a filter 36, and a two-dimensional imaging element 37, these components being arranged on an observation optical axis L1. Infrared illumination light sources 31 are used as an observation light source. An image of the examinee's eye illuminated by the infrared illumination light sources 31 is formed on the two-dimensional imaging element 37 via an optical path from the beam splitter 32 to the filter 36. Herein, the filter 36 has the property of transmitting the light from the light sources 31 and the light from an alignment light source 41 and not transmitting the light from a light source 61 mentioned later for detecting corneal deformation and visible light. The image formed on the two-dimensional imaging element 37 is displayed on a monitor 38.

The alignment optical system 40 has the light source 41 and a projection lens 42. In the present embodiment, the alignment optical system 40 shares the configuration from the beam splitter 32 to the two-dimensional imaging element 37 arranged on the optical axis L1 with the observation optical system 30. Infrared light projected from the alignment infrared light source 41 via the projection lens 42 is reflected by the beam splitter 32 and then projected to the examinee's eye E from a front direction. A corneal luminescent spot (a corneal reflection image) formed at a corneal vertex by the light source 41 is formed on the two-dimensional imaging element 37 via the beam splitter 32 to the filter 36. Even though the details thereof will be mentioned later, this corneal luminescent spot is utilized for alignment detection in up-and-down and right-and-left directions.

The fixation optical system 50 includes a visible light source (a fixation lamp) 51 and a projection lens 52. In the present embodiment, the fixation optical system 50 shares the components from the beam splitter 34 to the beam splitter 32 arranged on the optical axis L1 with the observation optical system 30 and others. Upon turn-on of the visible light source 51, a fixation target is presented to the examinee's eye E from front. Thus, the visual line of the examinee's eye E is fixed in a direction toward a fixation point located in the front direction. In this case, the optical axis L1 is used as a fixation optical axis. The visible light source 51 may include any light sources such as an LED and a laser, for example. As the visible light source 51, furthermore, there may be used a pattern light source such as a point light source, a slit light source, and a ring light source as well as a two-dimensional display such as a liquid crystal display.

The corneal deformation detecting optical system 60 is used to detect a deformed state of the cornea Ec. This optical system 60 includes a light projecting optical system 60a and a light receiving optical system 60b. The light projecting optical system 60a is arranged to irradiate illumination light toward the cornea Ec of the eye E from an oblique direction. The light projecting optical system 60a includes a light source 61, a dichroic mirror 62, and a projection lens 63. On the other hand, the light receiving optical system 60b is arranged to receive, by a photodetector 67, corneal reflection light from the light projecting optical system 60a. The light receiving optical system 60b includes a lens 64, a beam splitter 65, and a pinhole plate 66 in addition to the photodetector 67. In the present embodiment, the corneal deformation detecting optical system 60 is placed so that an amount of light received by the photodetector 67 is maximum when the examinee's eye E is in a predetermined deformed state (e.g., a flattened state). Accordingly, deformation of the examinee's eye E is detected based on a light receiving signal representing the amount of light received (light receiving amount) output from the photodetector 67.

The above deformation of the examinee's eye E is carried out by blowing a predetermined mount of compressed fluid (e.g., compressed air) from a nozzle 6 of the intraocular measuring unit 10 toward the examinee's eye E. This compressed fluid is supplied from a fluid supply unit 10a (see FIG. 4). In the ophthalmic measurement apparatus 1 in the present embodiment, the intraocular pressure is measured based on a deformation amount of the examinee's eye E detected by use of the photodetector 67 when the compressed fluid is blown onto the eye E.

The first working distance detecting optical system 70 is utilized for alignment (rough adjustment) in a working distance direction (a Z direction). The first working distance detecting optical system 70 shares the light projecting optical system 60a with the corneal deformation detecting optical system. This optical system 70 includes a light receiving optical system 70b. This light receiving optical system 70b includes a condensing lens 72 and a position imaging element 73. Light emitted from the light projecting optical system 60a forms an index image on the cornea Ec. Corneal reflection light from the light projecting optical system 60a enters the position detecting element 73 via the components from the lens 64 to the condensing lens 72. A light receiving position on the position detecting element 73 varies according to a positional relationship between the measuring unit and the examinee's eye E (the cornea Ec) in the working distance direction (the Z direction). Accordingly, the information on the working distance is obtained based on an output signal from the position detecting element 73. The output signal from the position detecting element 73 in the present embodiment is utilized for alignment (rough adjustment) in the working distance direction (the Z direction). The magnification of the light receiving optical system 70b of the first working distance detecting optical system is not as large as that of a light receiving optical system 80b mentioned later. Thus, a distance detection range of the position detecting element 73 in the Z direction is wider than that of a two-dimensional light receiving element 87. The distance detection range of the position detecting element 73 is for example a range of ±3 to 4 mm from an alignment reference position.

The corneal thickness measuring optical system 80 (80a, 80b) is used to measure the corneal thickness of the examinee's eye E. This optical system 80 includes a light projecting optical system 80a and a light receiving optical system 80b.

The light projecting optical system 80a is used to project measurement light (illumination light) for measuring the corneal thickness from an oblique direction with respect to the optical axis direction of the observation optical system 30. In the present embodiment, the light projecting optical system 80a includes a measurement light source 81, a condensing lens 82, a light limiting member 83, a concave lens 84, the beam splitter 65, a beam splitter 71, and the lens 64. These components are placed on an optical axis L2 which is a light projection optical axis. The optical axis L2 is inclined with respect to the optical axis L1 which is the fixation optical axis. Thus, the light projecting optical system 80a irradiates illumination light (measurement light) toward the examinee's eye E from an oblique direction with respect to the fixation optical axis. As the measurement light source 81, there is used a visible light source or an infrared (including near infrared) light source; e.g., an LED, a laser, or the like. The condensing lens 82 condenses the light emitted from the measurement light source 81.

The light limiting member 83 is placed on an optical path of the light projecting optical system 80a and limits the light (measurement light) emitted from the measurement light source 81. In the present embodiment, the light limiting member 83 is disposed in a position conjugated with a posterior surface of the cornea Ec during measurement of the corneal thickness. In the present embodiment, the light limiting member 83 is a pinhole plate, but is not limited thereto. For example, a slit plate or the like may also be used as the light limiting member 83. This light limiting member 83 allows a part of the light emitted from the measurement light source 81 to pass through and blocks a remaining part of the light. As a result, the light projecting optical system 80a projects a predetermined pattern of light (a spot light beam) to the cornea Ec.

In the present embodiment, the light receiving optical system 80b has the two-dimensional light receiving element 87. The light receiving optical system 80b is arranged to receive, by the two-dimensional light receiving element 87, the measurement light emitted from the light projecting optical system 80a and reflected (specular reflection) by a corneal anterior surface Ec1 (a corneal front surface, a corneal epithelium) and a corneal posterior surface Ec2 (a corneal back surface, a corneal endothelium) of the examinee's eye E. In the present embodiment, an optical axis L3 of the light receiving optical system 80b is placed nearly symmetrical to the optical axis L2 of the light projecting optical system 80a with respect to the observation optical axis (the optical axis L1) of the observation optical system 30. In the present embodiment, furthermore, the light receiving optical system 80b includes the lens 63, the dichroic mirror 62, a light receiving lens 85, and the two-dimensional light receiving element 87 on the optical axis L3. In the present embodiment, even though the details will be mentioned later, a detailed alignment state in the Z direction with respect to the examinee's eye E is detected by the light receiving optical system 80b in the present embodiment.

In the present embodiment, the two-dimensional light receiving element 87 receives reflection light from each of the corneal anterior surface and the corneal posterior surface. As the two-dimensional light receiving element 87, there can be used a two-dimensional imaging element such as a two-dimensional area sensor and a CCD.

The light receiving optical system 80b of the corneal thickness measuring optical system 80 performs imaging with high magnification with respect to the first working distance detecting optical system 70. Accordingly, in the present embodiment, the distance detection range of the two-dimensional light receiving element 87 in the Z direction is narrower than that of the position detecting element 73. For instance, it is a range of ±1 mm from the alignment reference position.

The light emitted from the measurement light source 81 is condensed by the condensing lens 82 and illuminates the light limiting member 83 from behind. The light from the measurement light source 81 is limited by the light limiting member 83 and then is imaged (condensed) in a position near the cornea Ec by the lens 64. In the present embodiment, specifically, a pinhole image is formed near the cornea Ec. At that time, the light from the measurement light source 81 is formed near an intersection point with a visual axis on the cornea Ec.

When the measurement light is projected onto the cornea Ec by the light projecting optical system 80a, the reflection light of the measurement light by the cornea Ec travels in a direction symmetrical to the projection light beam with respect to the optical axis L1. The light receiving lens 85 forms an image of the reflection light on a light receiving plane of the two-dimensional light receiving element 87.

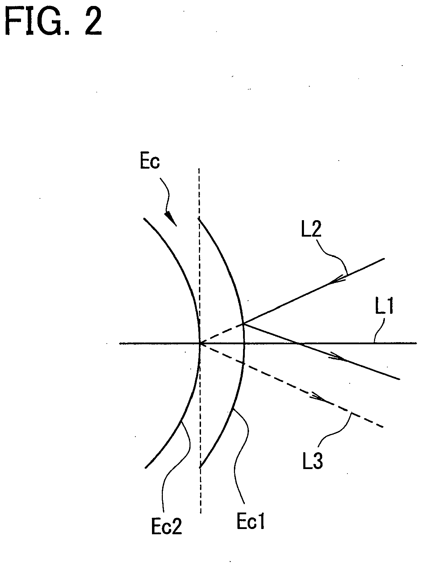

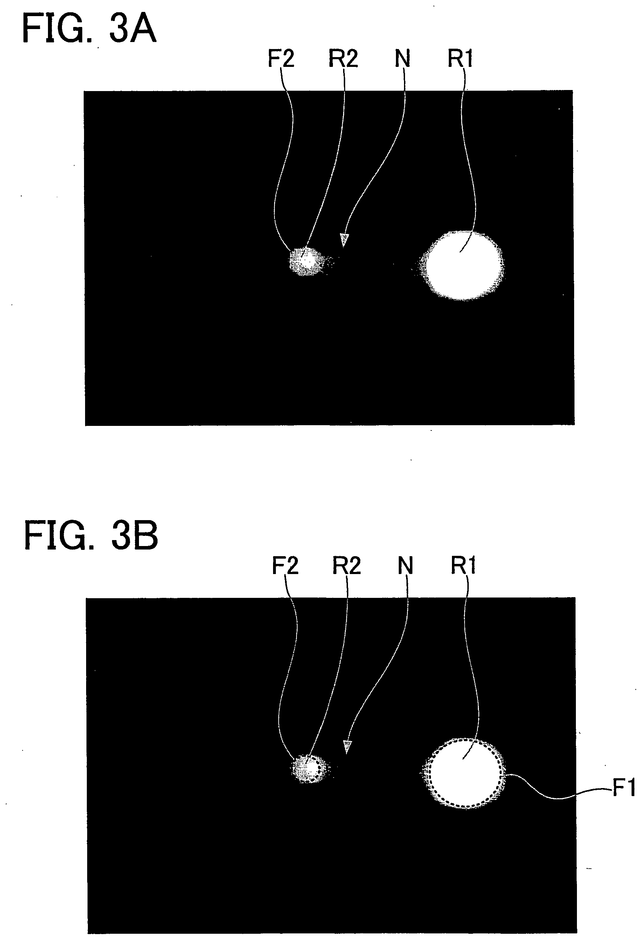

As shown in FIG. 2, a part of the measurement light irradiated from the light projecting optical system 80a to the cornea Ec is reflected by the corneal anterior surface Ec1. This forms, as shown in FIG. 3A, a first cornea reflection image R1 (hereinafter, simply referred to as "first reflection image R1") on the two-dimensional light receiving element 87. Another part of the measurement light from the light projecting optical system 80a is reflected by the corneal posterior surface Ec2. This forms, as shown in FIG. 3A, a second cornea reflection image R2 (hereinafter, simply referred to as "second reflection image R2" on the two-dimensional light receiving element 87. In the present embodiment, the first cornea reflection image R1 is a circular reflection image. The second cornea reflection image R2 is a circular reflection image smaller than the first cornea reflection image R1. The reflection light (see a solid line) by the corneal anterior surface and the reflection light (see a broken line) by the corneal posterior surface are different in reflection optical path and therefore two reflection images R1 and R2 are formed fully or partially away from each other. For instance, in FIG. 3A, the second reflection image R2 is fully formed away from the first reflection image R1. As the corneal thickness is large, the reflection optical paths of two reflection images are more separated. Accordingly, two reflection images are formed in the positions more spaced apart on the two-dimensional light receiving element 87 as the corneal thickness is larger. The ophthalmic measurement apparatus 1 of the present embodiment is therefore arranged to measure the corneal thickness based on the positional relationship between the first reflection image R1 and the second reflection image R2 on the two-dimensional light receiving element 87.

In the present embodiment, for example, as shown in FIG. 1, the light projecting optical system 60a and the light receiving optical system 80b share the optical axis L3 with each other, and the light projecting optical system 80a, the light receiving optical system 60b, and the light receiving optical system 70b share the optical axis L2 with one another. Needless to say, they are not limited thereto.

It is to be noted that the lens 64 shared among the light receiving optical systems 60b and 70b and the light projecting optical system 80a is placed in a position to make the reflection light emitted from the light source 61 and reflected by the cornea Ec condense on a center of a hole of the pinhole plate 66 and make the measurement light emitted from the measurement light source 81 condense on the anterior surface and the posterior surface of the cornea Ec.

A control system of the ophthalmic measurement apparatus 1 will be explained below. The ophthalmic measurement apparatus 1 of the present embodiment has a control unit 100 for executing control of the entire apparatus and calculation of measurement results.

In the present embodiment, the control unit 100 is connected to the fluid supply unit 10a, the XYZ drive unit 20, the operating unit 21, a storage device 105, the various light sources 31, 41, 51, 61, and 81, the various light receiving elements 37, 67, 73, 87, and others.

Furthermore, the control unit 100 includes a CPU 101, a ROM 102, and a RAM 103. The CPU 101 is a processing device (a processor) to execute various processings (e.g., control processing, arithmetic processing, etc.) related to the ophthalmic measurement apparatus 1. The ROM 102 is a nonvolatile storage device in which control programs, fixed data, and others are stored. The RAM 103 is a rewritable volatile storage device. This RAM 103 stores temporary data to be used by the ophthalmic measurement apparatus 1 in photographing and measuring the examinee's eye E.

The storage device 105 is a rewritable nonvolatile storage device. In the present embodiment, the storage device 105 stores at least a program to execute an anterior segment measurement processing by the control unit 100. The storage device 105 may also save an image captured by the ophthalmic measurement apparatus 1 and measurement results obtained by the ophthalmic measurement apparatus 1 and others.

Operations of the apparatus configured as above will be explained.

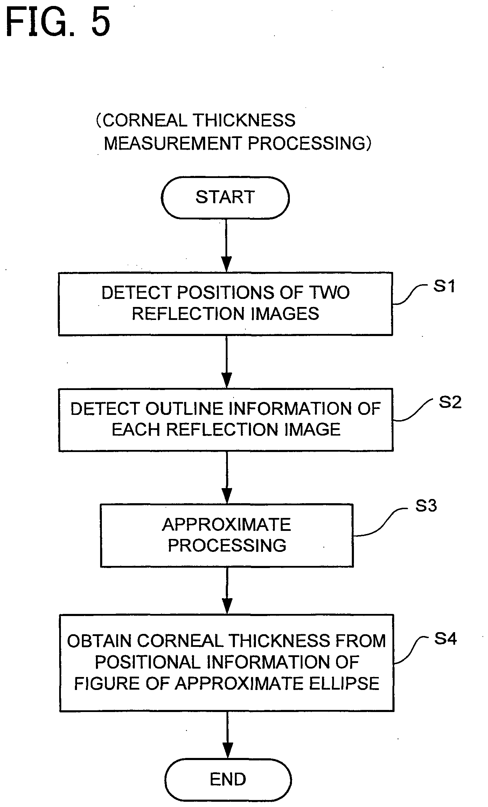

The following explanation will be given mainly to the operation of measuring the corneal thickness. In the present embodiment, in the ophthalmic measurement apparatus 1, the CPU 101 (the control unit 100) executes a corneal thickness measurement processing shown in FIG. 5 to measure the corneal thickness.

In the present embodiment, when the corneal thickness is to be measured, the measuring unit of the ophthalmic measurement apparatus 1 is subjected to alignment (positional adjustment) in advance. This alignment is performed after having the examinee's eye E positioned to face the measuring unit and having an examinee gaze at a fixation target. The alignment in X and Y directions (up-and-down and right-and-left directions) in the present embodiment may be performed by operation of the operating unit 21 (e.g., a joystick) by an examiner based on alignment information displayed on a monitor 38. For instance, the examiner manipulates the operating unit 21 so as to bring a corneal reflection image (a luminescent spot) formed by the light source 41 into a predetermined position relative to a reticle not shown displayed on the monitor 38. The CPU 101 may also automatically perform alignment adjustment in the up-and-down and right-and-left directions based on the alignment information obtained as an imaging result of the two-dimensional imaging element 37. In this case, the CPU 101 controls the XYZ drive mechanism 20 so that the corneal luminescent spot by the light source 41 is formed in an alignment position (e.g., an intersection point of the optical axis L1 and an imaging plane of the two-dimensional imaging element 37) set on the two-dimensional imaging element 37 in advance. In this way, the CPU 101 moves the measuring unit to an alignment completion position in the X and Y directions.

Successively, the alignment in the Z direction (a back-and-forth direction) will be performed. In the present embodiment, as described above, the alignment in the Z direction is automatically performed in such a manner that the CPU 101 controls the operation of the XYZ drive mechanism 20 based on working distance information obtained from the position detecting element 73 and the two-dimensional light receiving element 87.

When the corneal reflection light is detected first by the position detecting element 73, the CPU 101 causes the measuring unit to move in the Z direction based on the working distance information obtained from the position detecting element 37. In the present embodiment, consequently, the measuring unit is moved to a range where the reflection light (the first reflection image R1 or the second reflection image R2) of the light from the measurement light source 81 is detected by the two-dimensional light receiving element 87. Upon completion of the movement, the CPU 101 further minutely adjusts the positional relationship between the measuring unit and the examinee's eye E in the Z direction based on the light receiving signal from the two-dimensional light receiving element 87. More concretely, for example, the positional relationship is adjusted so that the corneal posterior surface of the examinee's eye E and the two-dimensional light receiving element 87 are optically conjugated with each other. In the present embodiment, for example, when the second reflection image R2 is formed at the center of the two-dimensional light receiving element 87, this light receiving element 87 is conjugated with the corneal posterior surface of the examinee's eye E. As the result of the detailed alignment adjustment, the second reflection image R2 formed on the two-dimensional light receiving element 87 becomes clearest. The light emitted from the light projecting optical system 80a and reflected by the corneal posterior surface is weaker than the light reflected by the corneal anterior surface, so that the second reflection image R2 is apt to be smaller and more obscure than the first reflection image R1. In the present embodiment, since the second reflection image R2 is made clearer by the detailed alignment adjustment, the position, the shape, and others of the second reflection image R2 can be easily detected.

In the present embodiment, after the above alignment adjustment, the corneal thickness measurement processing shown in FIG. 5 is executed by the CPU 101 to measure the corneal thickness. The corneal thickness measurement processing may be triggered by completion of the adjustment. The corneal thickness measurement processing may also be started according to an instruction from an examiner.

Herein, the corneal thickness measurement processing will be explained referring to FIG. 5. In the corneal thickness measurement processing of the present embodiment, the CPU 101 two-dimensionally detects the positional information of the first reflection image R1 and the second reflection image R2 and obtains the corneal thickness by use of the two-dimensionally detected positional information. To more concrete, based on the obtained positional information of the first reflection image R1 and the second reflection image R2, the corneal thickness is determined from a distance between the reflection images.

The CPU 101 first obtains a picture image including the first reflection image R1 and the second reflection image R2 based on a light receiving signal from the two-dimensional light receiving element 87 (S1). In this processing, for example, the picture image shown in FIG. 3A is obtained as image data.

The CPU 101 successively detects outline information of the first reflection image R1 and the second reflection image R2 included in the picture image obtained in the processing in S1 (S2). In this corneal thickness measurement processing, the corneal thickness is calculated by use of the positional information of the reflection images R1 and R2 defined based on the outline information, even though the details thereof will be described later.

The outline information is for example based on luminance gradient information of the first reflection image R1 and the second reflection image R2. The positional information of the edge of each of the reflection images R1 and R2 may be detected as the outline information. At that time, as a method to determine the position of the edge, it is conceived to use a luminance distribution of each reflection image R1 and R2 in a meridian direction. For instance, it may be arranged to perform a binarization processing on the image obtained by the processing in S1 and detect a boundary portion of a resultant image as the edge. As another alternative, furthermore, in the luminance distribution of each reflection image R1 and R2, a region of a certain luminance range having predetermined upper and lower limits may be detected as the edge of each reflection image R1 and R2.

The CPU 101 executes an approximate processing (fitting processing) on the outline information (e.g., the edge) of each of the reflection images R1 and R2 detected by the processing in S2 (S3). In the approximate processing (S3), for example, straight line, curved line, figure, and others may be fitted to the outline of each of the reflection images R1 and R2. The figure may include for example a combination of straight lines and curved lines as well as curved figures such as ellipse and circle, and rectilinear figures such as polygon. In the present embodiment, the approximate processing (S3) is explained as being performed by setting of an approximate figure (figure fitting) to the outline of each of the reflection image R1 and R2. One example is explained below as a case where approximate ellipses F1 and F2 (see FIG. 3B) are set respectively to the first reflection image R1 and the second reflection image R2.

Subsequently, the CPU 101 determines the corneal thickness from the positional information of each of the two-dimensionally detected reflection images R1 and R2 (S4). In the present embodiment, the corneal thickness is obtained by use of the positional information of each of the reflection images defined based on the outline information having been subjected to the approximate processing. For instance, the CPU 101 can also determine the center-to-center distance between the approximate ellipses F1 and F2 and obtain the corneal thickness based on the calculated center-to-center distance. For example, the center-to-center distance between the approximate ellipses F1 and F2 is converted to a measurement value of the corneal thickness by use of at least one of an arithmetic expression and a table. The obtained measurement value is stored in the storage device 105 or the like. In the case of using the arithmetic expression, this arithmetic expression may be created by for example optical simulation or the like in consideration of difference in refractive index between air and a cornea, difference in corneal curvature, and others. In the case of using the table, this table may be created by for example calibration using a known eye (e.g., a schematic eye) having different thickness.

The corneal thickness measurement (the processings in S1 to S4) may be repeated several times to obtain the measurement value of the corneal thickness from an average value, a central value, and others in each measurement.

In the corneal thickness measurement processing of the present embodiment as above, the positional information of the first reflection image R1 and the second reflection image R2 is two-dimensionally detected and the corneal thickness is measured by use of the two-dimensionally detected positional information. In the present embodiment, since the measurement is performed in consideration of the two-dimensional positional information of each of the reflection images R1 and R2, it is easier to control measurement errors of the corneal thickness resulting from corneal abnormality, extraneous matters to the cornea, noise N, and others as compared with a conventional art in which the corneal thickness is measured based on one line (one-dimensional) luminance information.

In the present embodiment where the approximate ellipses F1 and F2 are set, for instance, even when part of each of the reflection images R1 and R2 is uneven or irregular under the influence of corneal abnormality and other reasons, the influence of unevenness is dispersed throughout the approximate ellipses F1 and F2. As a result, the measurement value of the corneal thickness obtained from the positional relationship between the approximate ellipses F1 and F2 contains less measurement errors resulting from the corneal abnormality and others.

The approximate processing (S3) is performed on parts of the outlines (the edges) of the first reflection image R1 and the second reflection image R2 after removing a corneal inside portion therefrom. Thus, the set position of the approximate figure is less likely to be influenced by the noise N. This can obtain a more accurate measurement value of corneal thickness by the corneal thickness measurement processing.

The present disclosure is explained along the above embodiment, but is not limited thereto and may be variously modified.

For instance, the approximate processing (S3) does not necessarily require the outline information (e.g., the edge) of the entire circumference of each of the reflection images R1 and R2. As an alternative, fitting may be performed by use of the outline information of part of each of the reflection images R1 and R2.

For instance, when the cornea is abnormal or extraneous matters such as dust stick to the corneal anterior surface, each boundary of the first reflection image R1 and the second reflection image R2 may be partially vague. Such a vague boundary area does not need to be used for fitting. The vague boundary area exhibits a more gradual luminance change (luminance gradient) in the meridian direction of the reflection image than a clear boundary area. Therefore, the approximate processing may be performed in consideration of the degree of luminance change. For instance, when the degree of luminance change (e.g., luminance gradient) in the meridian direction of an image is larger than a predetermined threshold, this condition may be one of conditions for detecting the edge to be used for fitting. More concretely, when the luminance change degree in the median direction of the image is smaller than the predetermined threshold, it may be arranged not to detect the edge from the relevant region. As a result, the outlines of the reflection images R1 and R2 can undergo more preferable fitting and a more preferable measurement value of corneal thickness can be obtained.

As shown in FIG. 3A, furthermore, there is a case where a strip-shaped noise N occurs between the first reflection image R1 and the second reflection image R2 on the two-dimensional light receiving element 87. One conceivable example of the causes for this noise N is noise light directed in the optical axis L3 direction caused by scattering of the light from the light projecting optical system 80a inside the cornea Ec (i.e., between the anterior surface and the posterior surface). The corneal inside portion (a left portion of the first reflection image R1 and a right portion of the second reflection image R2 in FIG. 3) of each of the first reflection image R1 and the second reflection image R2 overlaps the noise N and thus is apt to have a vague boundary. Therefore, the approximate processing may be performed after removing noise component (noise N) occurring between the first reflection image R1 and the second reflection image R2. For example, the edge from which the corneal inside portion has been removed may be used for the fitting. This can perform the fitting with reduced influence of noise component, so that a more preferable measurement value of corneal thickness can be obtained.

Furthermore, the noise component can also be removed through the use of a difference between the noise component and the luminance information (luminance value and luminance gradient) of each of the reflection images R1 and R2. It is to be noted that the noise component may be removed from both of the first reflection image R1 and the second reflection image R2 or from either one of them.

When the light quantity output from the light source 81 or the gain of the two-dimensional light receiving element 87 is large, the first reflection image R1 and the second reflection image R2 may partially overlap. In this case, the boundary of each of the reflection images R1 and R2 is also less likely to be accurately specified. In the processing in S3, therefore, the edge from which the corneal inside portion has been removed may also be used for the fitting. This makes it possible to perform the fitting with reduced influence of the noise component, so that a more preferable measurement value of corneal thickness can be obtained.

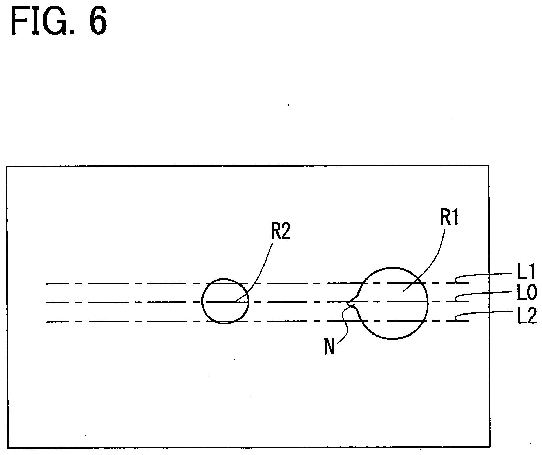

As one example of the method of measuring the corneal thickness based on the positional relationship between the first reflection image R1 and the second reflection image R2 and their two-dimensional shapes, the above embodiment shows the case where the approximate figures are set to the first reflection image R1 and the second reflection image R2. However, the method is not limited thereto. For instance, as shown in FIG. 6, the corneal thickness measurement processing is performed by detecting the positional information of the first cornea reflection image R1 and the second cornea reflection image R2 on at least two or more lines (L0, L1, L2, ....) along the corneal thickness direction and obtain the corneal thickness by use of the positional information on each of the lines. In FIG. 6, a plurality of lines (L0, L1, L2, ....) along the corneal thickness direction passing through each of the two reflection images R1 and R2 are set to an image obtained by use of the two-dimensional light receiving element 87. For instance, the CPU 101 determines a value of corneal thickness for each line from a distance between the center point of the first reflection image R1 and the center point of the second reflection image R2 on each line. Thereafter, the CPU 101 may obtain a final measurement value of corneal thickness in consideration of the value of corneal thickness on each line. An average value of corneal thicknesses on the lines is obtained as a measurement value. The center point of each of the reflection images R1 and R2 may be a position at which the luminance value on a line is peak. Furthermore, it may be an intermediate position between the edges of each of the reflection images R1 and R2.

Of the corneal thicknesses obtained one for each line, a corneal thickness having an error beyond a fixed value with respect to the corneal thicknesses obtained for other lines may be excluded when a final measurement value of corneal thickness (e.g., an average of the corneal thicknesses on the lines) is to be determined. The error in corneal thickness defined herein may also be an error to for example (a reference value of) a distribution of corneal thicknesses obtained on all the lines. In the example shown in FIG. 6, for instance, the noise N overlaps the first reflection image R1. Thus, on the line L0, the center point of the reflection image R1 is displaced toward the corneal inside as compared with the center points of the reflection image R1 on the lines L1 and L2. The corneal thickness obtained on the line L0 is therefore a smaller value than the corneal thickness obtained on each of the lines L1 and L2. At that time, for example, when the corneal thickness on the line L0 has an error more than a certain level from a reference value (an average value, a central value, etc.) of a distribution of corneal thicknesses on the lines L0 to L2, a final measurement value is calculated by use of the corneal thicknesses on the lines L1 and L2. Consequently, the ophthalmic measurement apparatus 1 can obtain a measurement value of the corneal thickness with reduced influence of noise N.

In the corneal thickness measurement processing, the CPU 101 may also be configured to determine a point of the center of gravity of each image of the first reflection image R1 and the second reflection image R2 and obtain corneal thickness from a distance between the gravity center points.

Moreover, for instance, the ophthalmic measurement apparatus 1 may be configured to correct a measurement value of intraocular pressure obtained based on the light receiving signal from the photodetector 67 of the corneal deformation detecting optical system 60, by use of the corneal thickness obtained as a result of the corneal thickness measurement processing. In this way, since the measurement value of intraocular pressure is corrected by use of accurate corneal thickness obtained by the corneal thickness measurement processing, a corrected value of the intraocular pressure can be easily obtained as an appropriate value.

In the above explanation, both the positional information of the first reflection image R1 and the second reflection image R2 are two-dimensionally detected; however, this disclosure is not limited thereto. For instance, even when the positional information of either the first reflection image R1 or the second reflection image R2 is two-dimensionally detected, a fixed level of effect can be obtained.

## Claims

1. An ophthalmic measurement apparatus (1) comprising: a light projecting optical system (80a) for projecting measurement light to an examinee's eye (E) from an oblique direction;
a light receiving optical system (80b) for receiving, by a light receiving element (87), a first cornea reflection image (R1) formed by the measurement light reflected by a corneal anterior surface (Ec1) of the examinee's eye and a second corneal reflection image (R2) formed by the measurement light reflected by a corneal posterior surface (Ec2) of the examinee's eye; and
an arithmetic processing unit (100) for obtaining corneal thickness of the examinee's eye based on positional information of the first cornea reflection image and the second cornea reflection image in a light receiving signal output from the light receiving element,
the light receiving optical system includes a two-dimensional light receiving element (87) as the light receiving element, and
the arithmetic processing unit is configured to two-dimensionally detect the positional information of the first cornea reflection image and the second cornea reflection image based on a light receiving signal from the two-dimensional light receiving element, and obtain the corneal thickness by use of the two-dimensionally detected positional information, **characterized in that** the arithmetic processing unit (100) is configured to detect positional information of the first cornea reflection image (R1) and the second cornea reflection image (R2) on at least two or more lines (L0, L1, L2) along a direction of thickness of a cornea, obtain an intermediary corneal thickness by use of the positional information on each of the lines, and obtain the corneal thickness as an average value of the intermediary corneal thicknesses on the lines.

2. The ophthalmic measurement apparatus according to claim 1, wherein a value of corneal thickness for each line is determined from a distance between the center point of the first reflection image R1 and the center point of second reflection image R2 on each line and the center point of each of the reflection images R1 and R2 is a position at which the luminance value on a line is peak.

3. The ophthalmic measurement apparatus according to claim 1, wherein a value of corneal thickness for each line is determined from a distance between the center point of the first reflection image R1 and the center point of second reflection image R2 on each line and the center point of each of the reflection images R1 and R2 is an intermediate position between the edges of each of the reflection images R1 and R2.

4. The ophthalmic measurement apparatus according to any one of claims 1 to 3, wherein the first cornea reflection image (R1) is a circular reflection image and the second cornea reflection image (R2) is a circular reflection image smaller than the first cornea reflection image.

## Patentansprüche

1. Ophthalmologische Messvorrichtung (1), umfassend: ein lichtprojizierendes optisches System (80a) zum Projizieren von Messlicht auf das Auge (E) eines Prüflings aus einer schrägen Richtung;
ein lichtempfangendes optisches System (80b) zum Empfangen, durch ein lichtempfindliches Element (87), eines ersten Hornhautreflexionsbildes (R1), das durch das von einer Hornhautvorderfläche (Ec1) des Auges des Prüflings reflektierten Messlichtes gebildet wird, und eines zweiten Hornhautreflexionsbildes (R2), das durch das von einer Hornhautrückfläche (Ec2) des Auges des Prüflings reflektierten Messlichtes gebildet wird; und
eine arithmetische Verarbeitungseinheit (100) zum Erhalten der Hornhautdicke des Auges des Prüflings basierend auf Positionsinformationen des ersten Hornhautreflexionsbildes und des zweiten Hornhautreflexionsbildes in einem Lichtempfangssignal, das von dem Lichtempfangselement ausgegeben wird,
das lichtempfangende optische System beinhaltet ein zweidimensionales lichtempfangendes Element (87) als das lichtempfangende Element, und
die arithmetische Verarbeitungseinheit konfiguriert ist, um die Positionsinformation von mindestens einem der ersten Hornhautreflexionsbilder und dem zweiten Hornhautreflexionsbild basierend auf einem Lichtempfangssignal des zweidimensionalen Lichtempfangselements zweidimensional zu erfassen, und die Hornhautdicke unter Verwendung der zweidimensional erfassten Positionsinformation zu erhalten, **dadurch gekennzeichnet, dass** die arithmetische Verarbeitungseinheit (100) konfiguriert ist, um Positionsinformationen des ersten Hornhautreflexionsbildes (R1) und des zweiten Hornhautreflexionsbildes (R2) auf mindestens zwei oder mehr Linien (L0, L1, L2) entlang einer Richtung der Dicke einer Hornhaut zu erfassen, die dazwischenliegende Hornhautdicke unter Verwendung der Positionsinformationen auf jeder der Linien zu erhalten, und die Hornhautdicke als Durchschnittswert der dazwischenliegenden Hornhautdicke auf den Linien zu erhalten.

2. Ophthalmologische Messvorrichtung nach Anspruch 1, wobei ein Wert der Hornhautdicke für jede Linie aus einem Abstand zwischen dem Mittelpunkt des ersten Reflexionsbildes R1 und dem Mittelpunkt des zweiten Reflexionsbildes R2 auf jeder Linie bestimmt wird, und der Mittelpunkt von jedem der Reflexionsbilder R1 und R2 eine Position ist, an der der Luminanzwert auf einer Linie Spitzenwert ist.

3. Ophthalmologische Messvorrichtung nach Anspruch 1, wobei ein Wert der Hornhautdicke für jede Linie aus einem Abstand zwischen dem Mittelpunkt des ersten Reflexionsbildes R1 und dem Mittelpunkt des zweiten Reflexionsbildes R2 auf jeder Linie bestimmt wird und der Mittelpunkt jedes der Reflexionsbilder R1 und R2 eine Zwischenposition zwischen den Kanten jedes der Reflexionsbilder R1 und R2 ist.

4. Ophthalmologische Messvorrichtung nach irgend einem der Ansprüche 1 bis 3, wobei das erste Hornhautreflexionsbild (R1) ein kreisförmiges Reflexionsbild und das zweite Hornhautreflexionsbild (R2) ein kreisförmiges Reflexionsbild ist, das kleiner als das erste Hornhautreflexionsbild ist.

## Revendications

1. Appareil de mesure ophtalmique (1) comprenant : un système optique de projection de lumière (80a) pour la projection de lumière de mesure jusqu'à un oeil d'une personne examinée (E) depuis une direction oblique ;
un système optique de réception de lumière (80b) pour la réception, par un élément de réception de lumière (87), d'une première image de réflexion de cornée (R1) formée par la lumière de mesure réfléchie par une surface antérieure cornéenne (Ec1) de l'oeil de la personne examinée et une deuxième image de réflexion de cornée (R2) formée par la lumière de mesure réfléchie par une surface postérieure cornéenne (Ec2) de l'oeil de la personne examinée ; et
une unité de traitement arithmétique (100) pour obtenir une épaisseur cornéenne de l'oeil de la personne examinée sur la base d'informations de position de la première image de réflexion de cornée et de la deuxième image de réflexion de cornée dans un signal de réception de lumière émis par l'élément de réception de lumière,
le système optique de réception de lumière comprend un élément de réception de lumière bidimensionnel (87) en tant qu'élément de réception de lumière, et
l'unité de traitement arithmétique est configurée pour détecter bidimensionnellement les informations de position de la première image de réflexion de cornée et de la deuxième image de réflexion de cornée sur la base d'un signal de réception de lumière de l'élément de réception de lumière bidimensionnel, et obtenir l'épaisseur cornéenne en utilisant les informations de position détectées bidimensionnellement, **caractérisé en ce que** l'unité de traitement arithmétique (100) est configurée pour détecter des informations de position de la première image de réflexion de cornée (R1) et de la deuxième image de réflexion de cornée (R2) sur au moins deux lignes (L0, L1, L2) ou plus le long d'une direction d'épaisseur d'une cornée, obtenir une épaisseur cornéenne intermédiaire en utilisant les informations de position sur chacune des lignes, et obtenir l'épaisseur cornéenne en tant que valeur moyenne des épaisseurs cornéennes intermédiaires sur les lignes.

2. Appareil de mesure ophtalmique selon la revendication 1, dans lequel une valeur d'épaisseur cornéenne pour chaque ligne est déterminée à partir d'une distance entre le point central de la première image de réflexion R1 et le point central de deuxième image de réflexion R2 sur chaque ligne et le point central de chacune des images de réflexion R1 et R2 est une position à laquelle la valeur de luminance sur une ligne est maximum.

3. Appareil de mesure ophtalmique selon la revendication 1, dans lequel une valeur d'épaisseur cornéenne pour chaque ligne est déterminée à partir d'une distance entre le point central de la première image de réflexion R1 et le point central de deuxième image de réflexion R2 sur chaque ligne et le point central de chacune des images de réflexion R1 et R2 est une position intermédiaire entre les bords de chacune des images de réflexion R1 et R2.

4. Appareil de mesure ophtalmique selon l'une quelconque des revendications 1 à 3, dans lequel la première image de réflexion de cornée (R1) est une image de réflexion circulaire et la deuxième image de réflexion de cornée (R2) est une image de réflexion circulaire plus petite que la première image de réflexion de cornée.
